## Europäisches Patentamt

## European Patent Office

## Office européen des brevets

(19)

(11) Veröffentlichungsnummer: **0 150 422**

**B1**

(12) # EUROPÄISCHE PATENTSCHRIFT

(45) Veröffentlichungstag der Patentschrift:
10.05.89

(21) Anmeldenummer: 84115651.6

(22) Anmeldetag: 17.12.84

(51) Int. Cl.⁴: **A 61 K 6/08,** A 61 C 9/00

(54) Verfahren zur Herstellung von Gipsmodellen für die Zahntechnik.

(30) Priorität: 30.12.83 DE 3347646

(43) Veröffentlichungstag der Anmeldung:
07.08.85 Patentblatt 85/32

(45) Bekanntmachung des Hinweises auf die Patenterteilung:
10.05.89 Patentblatt 89/19

(84) Benannte Vertragsstaaten:
CH DE FR GB LI

(56) Entgegenhaltungen:
EP-A-0 013 354
EP-A-0 090 493
WO-A-81/01959
GB-A-2 018 666

(73) Patentinhaber: ESPE Stiftung & Co Produktions-
und Vertriebs KG, D-8031 Seefeld (DE)

(72) Erfinder: Hübner, Heijo, Dr., Moosbichlweg 16,
D-8031 Wörthsee (DE)

(74) Vertreter: Abitz, Walter, Dr.- Ing., Abitz, Morf,
Gritschneder, Freiherr von Wittgenstein
Postfach 86 01 09, D-8000 München 86 (DE)

**Beschreibung**

Die Erfindung betrifft ein Verfahren zur Herstellung von Gipsmodellen für die Zahntechnik durch Behandeln eines in bekannter Weise erhaltenen Gipsmodells mit polymerisierbaren Estern der Arcyl- und/oder Methacrylsäure und Polymerisationsinitiatoren sowie Polymerisieren der Monomeren.

An Gipsmodellen für die Zahntechnik, z. B. an Modellen von Gebissen, die die Eingliederung von Zahnersatz oder kieferorthopädischen Apparaturen im zahntechnischen Labor ermöglichen, werden zahlreiche Arbeitsgänge durchgeführt. Die daraus resultierende Beanspruchung bewirkt einen Abrieb auf der Gipsoberfläche, wodurch die Passgenauigkeit des Werkstücks im Mund entscheidend beeinträchtigt werden kann. Bei relativ festsitzenden Werkstücken kann es auch vorkommen, dass der zu überkronende Zahnstumpf bei der Abnahme abbricht. Dadurch kann das gesamte Gebissmodell unbrauchbar werden, so dass der Arbeitsvorgang der Abdrucknahme und des Ausgiessens wiederholt werden muss. Zum Einpassen in das Gebiss wird das Gipsmodell häufig in einen sogenannten Artikulator eingebaut, der die Bewegung des Kiefergelenks simuliert, wobei das Gipsmodell mit dem Gipsmodell des Gegenkiefers in Kontakt kommt. Beim unvorsichtigen Öffnen und Schliessen des Artikulators kann im Gegenbiss Abrasion erzeugt werden; manchmal brechen sogar kleinere Teile des Antagonistenzahnes ab, durch die Passgenauigkeit des Werkstücks im Mund gefährdet ist.

Die Eigenschaften und Probleme handelsüblicher dentaler Modellwerkstoffe sind in Deutscher zahnärztlicher Zeitung, Bd. 32, (1977) S. 937 - 941 und S. 942 - 944 beschrieben.

Seit einiger Zeit sind die sogenannten Superhartgipse bekannt, welche verbesserte Abrasionsfestigkeit und/oder verbesserte Druckfestigkeit und Biegefestigkeit aufweisen. Meist ist jedoch nur eine der genannten Eigenschaften verbessert, während die anderen Eigenschaften unbefriedigend sind; ausserdem sind diese Gipse sehr kostspielig und häufig schwierig zu verarbeiten.

Man hat versucht, durch Auftragen von trocknenden Lacken auf Lösungsmittelbasis die Eigenschaften von Gipsmodellen zu verbessern (DE-A-3 009 755). Daraus ergibt sich jedoch nur eine geringfügige Verbesserung der Eigenschaften; ausserdem besteht die Gefahr einer Dimensionsveränderung des Modells durch das Auftragen des Lacks und das rasche Verdunsten des Lösungsmittels, bevor die Lösung in den Gips eingedrungen ist.

Man hat auch versucht, die Eigenschaften von Gipsmodellen mit Cyanacrylaten zu verbessern (Journ. Prost. Dent., Bd. 49, (1983) S. 639). Die rasche Erhärtung mit Wasser macht diese Anwendung jedoch unsicher, da das Eindringen des Cyanacrylats in den Gips unkontrollierbar wird. Überdies ist der Umgang mit Cyanacrylaten nicht ungefährlich.

Aus der EP-A-0 013 354 ist es bekannt, die Oberfläche von Gipsmodellen mit selbstpolymerisierenden Massen zu behandeln. Hierbei kann es jedoch zu unkontrollierten Dimensionsveränderungen des Gipsmodells kommen; auch die physikalischen Eigenschaften werden nur geringfügig verbessert, da die rasch polymerisierende Masse nur wenig in den Gips eindringen kann.

Der Erfindung liegt demnach die Aufgabe zugrunde, das Verfahren zur Herstellung von Gipsmodellen für die Zahntechnik durch Behandeln eines Gipsmodells mit polymerisierbaren Estern der Acryl- und/oder Methacrylsäure und Polymerisationsinitiatoren und Polymerisieren der Monomeren so abzuändern, dass die physikalischen Eigenschaften der Gipsmodelle, insbesondere ihre Abrasionsbeständigkeit und ihre Biege- und Druckfestigkeit, verbessert werden, wobei das Verfahren einfach und zeit- und kostengünstig durchzuführen sein soll.

Diese Aufgabe wird erfindungsgemäss dadurch gelöst, dass man ein in bekannter Weise erhaltenes Gipsmodell mit

(a) mindestens einem photopolymerisierbaren Ester der Acryl- und/oder Methacrylsäure,
(b) mindestens einem Photopolymerisationsinitiator und
(c) gegebenenfalls mindestens einem Photopolymerisationsaktivator tränkt,

wobei (a), (b) und (c) im flüssigen oder gelösten Zustand vorliegen, und das getränkte Gipsmodell mit einem für die Initierung der Photopolymerisation durch den Photoinitiator geeigneten Licht bestrahlt.

Man kann die Bestandteile (a), (b) und (c) gleichzeitig oder nacheinander anwenden.

Nach einer bevorzugten Ausführungsform kann man die Bestandteile (b) und/oder (c) als Mischung mit mindestens einem Teil von (a) einsetzen.

Die Viskosität der Bestandteile (a), (b), (c), ihrer Mischungen oder Lösungen beträgt bevorzugt ≤ 5 Poise, insbesondere < 1 Poise.

Durch das erfindungsgemässe Verfahren erhält man in zeit- und kostengünstiger Weise Gipsmodelle mit starken Verbesserungen der Abrasionsfestigkeit, Druckfestigkeit und Biegefestigkeit, ohne dass die Dimension der Modelle nennenswert verändert wird. Eigenschaftsschwankungen durch verwendete Variationen im Verhältnis Gips/Wasser und in der Anmischtechnik, die in der Praxis häufig vorkommen, werden durch das erfindungsgemässe Verfahren praktisch eliminiert. Aus einer Mischung geringerer Qualität wird durch das erfindungsgemässe Verfahren ein hochabrasionsfestes, druck- und biegefestes Modell gewonnen.

Für das erfindungsgemässe Verfahren können Gipsmodelle aus beliebigen, dental verwendbaren Gipsarten eingesetzt werden. Die eingesetzten Gipsarten bestehen im wesentlichen aus Calciumsulfat; sie können Zusätze enthalten, die dem Gipsmodell günstigere Eigenschaften, z. B. besseres Abbindeverhalten oder grössere Härte, verleihen. Die grundlegende Chemie der Dentalgipse ist in "Ullmanns Encyclopädie der technischen Chemie", 4. Auflage, Band 10, (1975) S. 19 beschrieben. Der Gips darf jedoch - je nach

2

eingesetztem Photoinitiator (b) keine so starke Lichtabsorption im Bereich von 250 - 500 nm aufweisen, dass die Polymerisation beeinträchtigt wird. Bevorzugt hat der Gips nur geringe Lichtabsorption im genannten Bereich, besonders bevorzugt werden ungefärbte Gipse verwendet.

Als photopolymerisierbare Ester der Acryl- und Methacrylsäure kommen auch die sogenannten Urethanacrylate und -methacrylate in Betracht.

Die Urethanacrylate und -methacrylate werden in bekannter Weise durch Umsetzung von Isocyanaten mit Hydroxyalkylacrylaten oder -methacrylaten gewonnen. Es ist auch möglich, Hydroxylverbindungen mit einem Äquivalent eines Diisocyanats umzusetzen, und die erhaltenen Isocyanatverbindungen dann mit Hydroxyalkylacrylaten oder -methacrylaten zu den Urethanacrylaten oder -methacrylaten zur Reaktion zu bringen. Geeignete Produkte sind unter der Warenkennzeichnung "Genomer" im Handel.

Es können sowohl monofunktionelle als auch di- oder polyfunktionelle Acrylate und Methacrylate eingesetzt werden. Bevorzugt beträgt der Anteil an monofunktionellem Acrylat und/oder Methacrylat 0 - 70 Gew.-% des Bestandteils (a), besonders bevorzugte monofunktionelle Derivate sind Methylmethacrylat und Hydroxyethylmethacrylat.

Geeignete Photopolymerisationsinitiatoren sind alle für die Photopolymerisation von Derivaten der Acrylsäure und Methacrylsäure bekannten Substanzen, wie aromatische Monoketone, Thioxanthone, aromatische und aliphatische 1,2-Diketone, Benzoinether und Benzilketale.

Von den genannten Photoinitiatoren haben sich die 1,2-Diketone besonders bewährt. Zur Verhinderung einer klebrigen Oberfläche des Polymerisats, die durch die Inhibitorwirkung des Luftsauerstoffs entsteht, wird in bevorzugter Weise eine Kombination aus mehreren Photoinitiatoren eingesetzt, deren aktive Lichtabsorption bei unterschiedlichen Wellenlängen liegt. Besonders geeignet ist eine Kombination aus Benzophenon (aktive Absorption bei ca. 255 nm und ca. 345 nm), Benzildimethylketal (aktive Absorption bei ca. 360 nm) und Campherchinon (aktive Absorption bei ca. 460 nm) oder Phenanthrenchinon (aktive Absorption bei ca. 420 nm).

Die Photoinitiatoren werden in den üblichen Konzentrationen eingesetzt, das sind 0,01 - 3 Gew.-%, bezogen auf den Bestandteil (a).

In vorteilhafter Weise werden die Photoinitiatoren zusammen mit bekannten Photoaktivatoren eingesetzt. Geeignete Photoaktivatoren sind organische Amine, insbesondere tertiäre Amine, zyklische 1,3-Diketone, wie Barbitursäuren und 2-substituierte 1,3-Cyclopentandione und 1,3-Cyclohexandione, sowie organische Phosphite.

Gegebenenfalls können den Bestandteilen (a), (b), (c) oder ihren Mischungen zur Verringerung der Viskosität leichtflüchtige Lösungsmittel, wie Methylenchlorid, zugesetzt werden. Bevorzugt sind hydrophile Lösungsmittel, wie Aceton, die gleichzeitig eine höhere Eindringgeschwindigkeit auch bei frischen, feuchten Gipsmodellen ermöglichen. Die Viskosität der Gemische kann aber auch durch die Verwendung von monofunktionellen Methacrylaten in geeigneter Konzentration eingestellt werden. Die Verwendung hydrophiler Methacrylate (bis zu 50 Gew.-% des Bestandteils (a)) ist günstig, da auf diese Weise ein besseres Eindringen des Bestandteils (a) oder seiner Mischung mit (b) und/oder (c) in das feuchte Gipsmodell gewährleistet ist. Geeignete hydrophile Methacrylate sind die Hydroxyalkylmethacrylate, besonders 2-Hydroxyethylmethacrylat und 3-Hydroxypropylmethacrylat.

Um eine bessere Filmbildung zu erreichen oder gegebenenfalls die Viskosität anzuheben, können den Bestandteilen (a), (b) und/oder (c) lösliche Polymere zugesetzt werden.

Auch der Zusatz von oberflächenaktiven Substanzen zur Verbesserung des Eindringens von (a), (b) und/oder (c) in das Gipsmodell ist möglich. Geeignet sind z. B. die hydrophilen Sorbimacrogole.

Die Bestandteile (a), (b) und/oder (c) können auch mit geeigneten Farbstoffen gefärbt werden, die Farbstoffe müssen aber so ausgewählt werden, dass sie keine starke Eigenabsorption in dem Bereich aufweisen, in dem die verwendeten Photoinitiatoren ihre aktive Lichtabsorption haben. Bei der Verwendung von Photoinitiatoren für den sichtbaren Bereich (z. B. Campherchinon, aktive Absorption bei 400 bis 500 nm) dürfen die eingesetzten Farbstoffe nur geringe Eigenabsorption zwischen 400 und 500 nm aufweisen.

Es ist möglich, zusätzlich zum Photoinitiator noch (d) ein organisches Peroxid oder Hydroperoxid sowie (e) einen Aktivator hierfür, wie aromatische Amine oder Thioharnstoffderivate, einzusetzen, die vor der Verwendung miteinander gemischt werden. Die Konzentrationen an (d) und (e) müssen so gewählt werden, dass die Polymerisation nach dem Vermischen von (d), (e) und den anderen Bestandteilen erst nach einigen Minuten eintritt, um ein tiefes Eindringen des Bestandteils (a) in den Gips zu gewährleisten. Vorzugsweise soll die Polymerisation in diesem Fall frühestens 5 Minuten nach dem Vermischen von (d) und (e) mit den anderen Bestandteilen einsetzen. Auf diese Weise können auch tiefere Schichten ausgehärtet werden, die der Polymerisation durch Einwirkung des Lichts allein nicht mehr zugänglich sind.

Die Bestandteile (a), (b), (c), (d) und (e) oder ihre Mischungen miteinander können mit den üblichen Massnahmen ein- oder mehrmals auf das Gipsmodell aufgebracht werden: Aufsprühen, Eintauchen oder Auftragen mit einem Pinsel sind möglich. Bevorzugt wird das Gipsmodell 3 bis 15 Minuten eingetaucht, besonders geeignet sind Tauchzeiten von 7 bis 10 Minuten. Die Mindesttiefe für das Eindringen des Bestandteils (a) in das Gipsmodell beträgt vorzugsweise 0,1 mm, insbesondere mindestens 0,5 mm.

Zur Bestrahlung des getränkten Gipsmodells können die üblichen Lampen verwendet werden, soweit sie genügend Strahlung in dem Wellenlängenbereich abgeben, der der aktiven Lichtabsorption des eingesetzten Photoinitiators (b) entspricht. In Frage kommen Xenonstrahler, unter Nieder-, Mittel- und Hochdruck betriebene Quecksilberstrahler, Halogenlampen und Fluoreszenzlampen, sowie Kombinationen der genannten Strahler. Bevorzugt werden Fluoreszenzlampen eingesetzt, da diese nur eine geringe Wärmeentwicklung aufweisen und keiner aufwendigen Kühlungsmassnahmen bedürfen.

Die Bestrahlungszeiten können je nach Intensität der Lichtquelle wenige Minuten bis einige Stunden betragen. Mit Fluoreszenzlampen liegen typische Bestrahlungszeiten zwischen 15 Minuten und 1 Stunde. Zur Erzielung einer trockenen Oberfläche des Polymerisats muss die Inhibierung des Luftsauerstoffs überwunden werden. Dies kann durch die Anwesenheit von Strahlung im Bereich zwischen 250 und 370 nm zusammen mit einem Photoinitiator, dessen aktive Lichtabsorption im genannten Bereich liegt, erfolgen. Vorzugsweise wird als Photoinitiator ein Gemisch aus Benzophenon und Benzildimethylketal eingesetzt, als Lichtquelle dient dann eine Fluoreszenzlampe mit einer Emission bei ca. 250 nm, sowie eine zweite Fluoreszenzlampe mit einer Emission bei ca. 360 nm.

Die Sauerstoffinhibierung kann aber auch durch die Verwendung eines Schutzgases, wie Stickstoff, Kohlendioxid oder Argon, überwunden werden; die Schutzgases können unter normalem Druck oder Überdruck eingesetzt werden. Eine Polymerisation unter Vakuum zur Eliminierung des Luftsauerstoffs ist weniger geeignet, da sich durch Verdampfung von im Gips enthaltenem Wasser Blasen bilden und überdies einige Vakuumpumpen durch den anfallenden Wasserdampf geschädigt werden können. Die Einwirkung des Luftsauerstoffs kann auch durch Auftragen einer Schutzschicht aus z. B. Wachs oder Glycerin verringert werden.

Um eine gute Tiefenpolymerisation zu erreichen, ist zusätzlich zu den beiden oben genannten Lichtarten der Einsatz von sichtbarem Licht im Spektralbereich 400 - 500 nm günstig; zusammen mit einer entsprechenden Fluoreszenzlampe werden dann vorzugsweise 1,2-Diketone als Photoinitiatoren eingesetzt - besonders geeignet sind Campherichinon und Phenanthrenchinon, gegebenenfalls zusammen mit einem Amin als Photoaktivator.

**Beispiele**

Folgende photopolymerisieibare Zubereitungen werden hergestellt:

**Lösung A**

In einem Gemisch aus

5,00 g Methacrylsäure-methylester und
15,00 g Bis-hydroxymethyl-bicyclo[5.2.1.02,6]-decandiacrylat

werden

1,00 g Benzophenon
0,10 g Benzildimethylketal
0,06 g Campherchinon und
0,30 g Methyl-diethanolamin-dimethacrylat

gelöst.

**Lösung B**

In einem Gemisch aus

5,00 g Methacrylsäure-methylester und
15,00 g Bishydroxymethyl-bicyclo[5.2.1.02,6]-decandiacrylat

werden

1,00  g Benzophenon
0,10  g Benzildimethylketal und
0,004 g Phenanthrenchinon

gelöst.

**Lösung C**

In

10,5 g Lösung B

werden

0,20 g Sorbimacrogol-laurat (Warenkennzeichnung "Tween 20")

gelöst.

**Lösung D**

In einem Gemisch von

6,67 g Bishydroxymethyl-bicyclo]5.2.1.02,6]-decandiacrylat,
6,67 g Methacrylsäure-methylester und
6,67 g Methacrylsäure-2-hydroxyethylester

werden

1,00 g Benzophenon
0,10 g Benzil-dimethylketal
0,06 g Campherchinon und
0,30 g Methyl-diethanolamin-dimethacrylat

gelöst.

**Lösung E**

In

5,00 g Lösung D

werden

0,25 g Sorbimacrogol-laurat (Warenkennzeichnung "Tween 20")

gelöst.

**Lösung F**

In

20,00 g Bis-hydroxymethyl-bicyclo[5.2.1.0.2,6]-decandiacrylat

werden

1,00  g Benzophenon
0,10  g Benzildimethylketal und
0,004 g Phenanthrenchinon

gelöst.

**Lösung G**

In einem Gemisch von

10,00 g   trifunktionellem, aliphatischem Urethan/Polyester-Acrylat (theoretisches mittleres Molgewicht 1600, Warenkennzeichnung "Genomer T 1600" der Fa. Rahn) und

10,00 g   Methacrylsäuremethylester

werden

1,00 g Benzophenon
0,10 g Benzildimethylketal
0,06 g Campherchinon und
0,30 g Methyl-diethanolamin-dimethacrylat

gelöst.

Die erhaltenen Lösungen werden in eine lichtdichte, sauerstoffdurchlässige Flasche (z. B. schwarze Polypropylenflasche) gefüllt und sind darin haltbar.

**Beispiel 1**

Ein Meistermodell aus einem handelsüblichen Hartgips (Fa. Kerr, Warenkennzeichnung "Vel-Mix-Stone") für dentale Prothetikarbeiten wird wie üblich durch Ausgiessen des zahnärztlichen Abdrucks der Kieferpartie gewonnen. Nach bekannten Verfahren wird das Modell nach ca. 30 Minuten entformt und 24 Stunden aushärten gelassen.

Auf die Zahnstümpfe dieses Gipsmodells wird mit einem feinen Pinsel Lösung A aufgebracht. Nach kurzer Zeit (ca. 1 - 2 Minuten) ist die gesamte Lösung in den Gips eingedrungen, die zunächst glänzende Oberfläche ist wieder matt geworden. Der Lösungsauftrag wird so lange wiederholt (3 - 4 Mal), bis eine glänzende Schicht auf dem Zahnstumpf verbleibt. Diese Schicht wird mit einem fusselfreien, saugfähigen Tuch sorgfältig entfernt.

Das vorbehandelte Modell wird nun 20 Minuten in ein Belichtungsgerät gegeben, das 3 Leuchtstoffröhren enthält. Das Intensitätsmaximum der 3 Röhren liegt bei 255 nm, 360 nm kund 460 nm.

Nach dem Ende der Bestrahlung hat man ein Gipsmodell, dessen Stümpfe passgenau, abrasions- und bruchfest sind.

**Beispiel 2**

Lösung B wird in eine Schale gegossen und ein wie in Beispiel 1 erhaltenes Gipsmodell wird mit den Stümpfen und den Zähnen des Restgebisses 7 Minuten lang in diese Lösung eingetaucht.

Nach dem Entfernen aus der Lösung werden die eingetauchten Partien sorgfältig mit Pressluft von der überstehenden Flüssigkeitsschicht befreit. Anschliessend wird, wie in Beispiel 1 beschrieben, belichtet.

Man erhält ein Gipsmodell, dessen Zahn-, Stumpf- und Kieferpartien sehr abrasions- und bruchfest sind.

Bei Verwendung der Lösungen F und G anstelle von Lösung B werden vergleichbare Ergebnisse erhalten.

**Beispiel 3**

Ein nach Beispiel 1 gewonnenes Gipsmodell wird bereits 2 Stunden nach dem Entformen mit der Lösung C, wie in Beispiel 1 beschreiben, behandelt. Die Lösung dringt auch in den feuchten Gips gut ein. Nach 20-minütigem Bestrahlen mit der in Beispiel 1 genannten Lampe wird ein Modell erhalten, dessen Stümpfe hohe Biegefestigkeit und Abrasionsfestigkeit aufweisen.

**Beispiel 4** (Vergleichsversuch)

Aus einer Ritzhärteprüfung nach Martens wird der spezifische Ritzvolumenverlust pro 1 cm Ritzstrecke bestimmt.

Dazu wird ein handelsüblicher dentaler Hartgips (Warenkennzeichnung "Moldano", Fa. Bayer) in Platten von 25 mm x 25 mm x 2 mm gegossen. Nach der entsprechenden Alterung der Probekörper wird eine Stahlnadel mit einem Kegelwinkel von 90° unter einer Last von 10 N aufgesetzt. Diese Nadel wird über eine motorisch angetriebene Exzenterscheibe ca. 2 cm über den Gips gezogen.

Durch Ausmessung unter dem Messmikroskop wird die Ritzbreite der Stahlnadel bestimmt und aus dieser der spezifische Volumenverlust pro 1 cm Wegstrecke berechnet.

In der nachfolgenden Tabelle sind die Ritzvolumenverluste, für den unbehandelten Gipskörper und für die entsprechend Beispiel 1 mit den Lösungen A, D und E behandelten Probekörper aufgeführt. Jede Prüfung wurde an Gips durchgeführt, der vor der Messung 1 Stunde, 2 Stunden und 1 Woche bei Normklima (23 $\pm$ 1°C, 45 $\pm$ 5 % relative Luftfeuchtigkeit) gelagert worden war.

**Tabelle 1**

| Gipslagerung bei Normalklima | Behandlung | spezifischer Ritzvolumenverlust pro 1 cm Ritzstrecke $[mm^3 \times 10^{-3}]$ |
|---|---|---|
| 1 h | keine | 1140 |
| | Lösung A | 480 |
| | Lösung D | 300 |
| | Lösung E | 200 |
| 2 h | keine | 470 |
| | Lösung A | 150 |
| | Lösung D | 83 |
| | Lösung E | 76 |
| 1 Woche | keine | 225 |
| | Lösung A | 69 |
| | Lösung D | 51 |
| | Lösung E | 51 |

Zu jedem Zeitpunkt der Gipsaushärtung wird durch das erfindungsgemässe Verfahren eine Verbesserung der Abriebfestigkeit um mindestens das 3-fache erzielt.

Bereits 2 Stunden nach der Herstellung eines Gipsmodells werden Abriebfestigkeiten erhalten, die der des vollständig ausgehärteten Gipses weit überlegen sind. Auch im vollständig ausgehärteten Gips wird durch das erfindungsgemässe Verfahren der Abrieb auf 1/3 bis 1/4 reduziert.

Aus der Tabelle 1 ist ersichtlich, dass hydrophile Zusätze besonders bei frischen, feuchtem Gips wirksam sind, bei trockenem Gips sind die Unterschiede gering.

**Beispiel 5** (Vergleichsversuch)

Wie in Beispiel 4 beschrieben, wurden Probekörper aus einem handelsüblichen Hartgips (Warenkennzeichnung "Duroc", Fa. Ransom + Randolph) hergestellt und der spezifische Ritzvolumenverlust für den unbehandelten Gipskörper, für nach dem Stand der Technik behandelte Gipskörper und für einen erfindungsgemäss behandelten Gipskörper ermittelt (Tabelle 2).

Ausserdem wurden aus den hergestellten Testplatten Normkörper für die Biegeprüfung nach DIN 13922 von 2 mm x 2 mm x 25 mm ausgesägt; die Stützweite betrug 10 mm. Im Falle des erfindungsgemässen Verfahrens wurden diese Körper 7 Minuten in die Lösung A eingetaucht, der Überschuss, wie in Beispiel 2 beschrieben, entfernt und wie in Beispiel 1 beschrieben belichtet. Die Materialien nach dem Stand der Technik wurden nach den Angaben des Herstellers mit einem Pinsel aufgebracht und entsprechend getrocknet.

# EP 0 150 422 B1

**Tabelle 2**

| Härtungslösung | spezifischer Ritzvolumen-verlust pro 1 cm Ritzstrecke [mm³ x 10⁻³] | Biegefestigkeit [MPa] | |
|---|---|---|---|
| keine | 245 | 19,0 | |
| Polymethylmethacrylat in Aceton (Warenkennzeichnung "Almadent Gipsversiegelung") (Stand der Technik) | 235 | 19,2 | |
| Warenkennzeichnung "Bego Stumpflack 2000" (DE-A-3 009 755) | 220 | 18,8 | nicht masshaltig! |
| A (erfindungsgemäss) | 66 | 30,8 | |

Während die Verfahren nach dem Stand der Technik nur eine geringfügige Erhöhung der Abrasionsfestigkeit und keine Veränderung der Biegefestigkeit erbringen, wird durch das erfindungsgemässe Verfahren eine erhöhte Biegefestigkeit und eine stark verbesserte Abrasionsbeständigkeit erreicht.

**Beispiel 6** (Vergleichsversuch)

In der nachfolgenden Tabelle 3 sind der spezifische Ritzvolumenverlust (Bestimmung wie in Beispiel 4) und die Biegefestigkeit (Bestimmung wie in Beispiel 5) einer Reihe von handelsüblichen Hartgipsen aufgeführt, die einmal ohne Härtung, und zum anderen nach Härtung mit Lösung A (sh. Beispiele 4 und 5) untersucht wurden. Ausserdem wurde die Druckfestigkeit an Prüfkörpern der Abmessung 2 x 2 x 4 mm bestimmt, die analog den Biegefestigkeitsstäben nach Beispiel 5 behandelt wurden.

**Tabelle 3**

| Untersuchter Gips | spezifischer Ritz-volumenverlust pro 1 cm Ritstrecke [mm³ x 10⁻³] | Biegefestigkeit [MPa] | Druckfestigkeit [MPa] |
|---|---|---|---|
| Kaffir D* (Firma Kaffir) unbehandelt | 728 | 20,8 | 54,7 |
| erfindungsgemäss mit Lösung A | 88 | 30,8 | 123,3 |
| Moldano* (Firma Bayer) unbehandelt | 225 | 20,1 | 46,7 |
| erfindungsgemäss mit Lösung A | 69 | 28,5 | 76,0 |
| Vel-Mix-Stone* (Firma Kerr) unbehandelt | 487 | 27,9 | 84,3 |
| erfindungsgemäss mit Lösung A | 45 | 32,7 | 129,5 |
| Duroc* (Firma Ransom + Randolph) unbehandelt | 245 | 19,0 | 59,3 |
| erfindungsgemäss mit Lösung A | 66 | 30,8 | 86,2 |
| Begolith* (Firma Bego) unbehandelt | 51 | 29,0 | 110,5 |
| erfindungsgemäss mit Lösung A | 42 | 38,6 | 134,1 |

* Warenkennzeichnung

**Beispiel 7** (Vergleichsversuch)

Prüfkörper aus handelsüblichem Modellgips (Warenkennzeichnung "Moldano", Fa. Bayer) zur Messung des spezifischen Ritzvolumenverlustes pro 1 cm Wegstrecke, der Biegefestigkeit und der Druckfestigkeit (siehe Beispiel 6 wurden zunächst 24 h bei Normklima (siehe Beispiel 4) aufbewahrt und dann vor der Messung folgendermassen behandelt:

I     Keine weitere Vorbehandlung

II    Erfindungsgemäss mit Lösung A entsprechend Beispiel 6

III   Die Probekörper wurden 15 Sekunden in eine Lösung von 10 Gew.-% Benzoylperoxid und 90 Gew.-% Aceton eingetaucht. Nach 5 Minuten Trockenzeit wurden die Probekörper dann 100 Sekunden in eine Mononermischung aus 47 Gew.-% Bis-GMA (Reaktionsprodukt aus Bisphenol A und Glycidylmethacrylat) und 51 Gew.-% Triethylenglykol-dimethacrylat (enthaltend 2 Gew.-% N,N-Diethanol-p-toluidin) getaucht. Anschliessend wurden die Prüfkörper mit Aceton abgewischt (siehe EP-A-0 013 354, Beispiel 1).

IV   10 Tropfen des Monomergemischs aus III (enthaltend 2 Gew.-% N,N-Diethanol-p-toluidin) wurden mit 10 Tropfen dieses Monomerengemischs (enthaltend 2 Gew.-% Benzoyl-peroxid) vermischt. Diese Mischung wurde mit einem Pinsel auf die Probekörper aufgetragen. Nach 1 Minute wurde der Überschuss abgewischt (siehe EP-A-0 013 354, Beispiel 1, letzter Absatz).

Die Messergebnisse sind in Tabelle 4 wiedergegeben.

**Tabelle 4**

| Vorbehandlung | spezifischer Ritz-volumenverlust pro 1 cm Ritzstrecke $[mm^3 \times 10^{-3}]$ | Biegefestigkeit [MPa] | Druckfestigkeit [MPa] |
|---|---|---|---|
| I | 225 | 20,1 | 46,7 |
| II | 69 | 28,5 | 76,0 |
| III | 108 | 17,8 | 68,6 |
| IV | 121 | 20,3 | 58,6 |

Nur die Prüfkörper nach I und II waren maßhaltig. Die Biegefestigkeit wird nur durch das erfindungsgemässe Verfahren verbessert, gegenüber dem Verfahren der EP-A-0 013 354 werden Abrasionswiderstand und Druckfestigkeit nochmals deutlich verbessert.

**Patentansprüche**

1. Verfahren zur Herstellung von Gipsmodellen für die Zahntechnik durch Behandeln eines in bekannter Weise erhaltenen Gipsmodells mit polymerisierbaren Estern der Acryl- und/oder Methacrylsäure und Polymerisationsinitiatoren sowie Polymerisieren der Monomeren, dadurch gekennzeichnet, dass man das Gipsmodell mit

(a) mindestens einem photopolymerisierbaren Ester der Acryl- und/oder Methacrylsäure,

(b) mindestens einem Photopolymerisationsinitiator und

(c) gegebenenfalls mindestens einem Photopolymerisationsaktivator

tränkt, wobei (a), (b) und (c) im flüssigen oder gelösten Zustande vorliegen, und das getränkte Gipsmodell mit einem für die Initiierung der Photopolymerisation durch den Photoinitiator geeigneten Licht bestrahlt.

2. Verfahren nach Anspruch 1, dadurch gekennzeichnet, dass (b) und/oder (c) als Mischung mit mindestens einem Teil von (a) eingesetzt werden.

**Claims**

1. A process for producing plaster models for use in dentistry by treating a plaster model obtained in a manner known per se with polymerizable esters of acrylic and/or methacrylic acid and polymerization initiators, and polymerizing the monomers, characterized in that the plaster model is impregnated with

(a) at least one photopolymerizable ester of acrylic and/or methacrylic acid,

(b) at least one photopolymerization initiator, and

(c) optionally at least one photopolymerization activator,

the components (a), (b) and (c) being present in liquid or dissolved state, and the impregnated plaster model is irradiated with light suited for initiation of the photopolymerization by the photoinitiator.

2. Process according to claim 1, characterized in that (b) and/or (c) are employed in the form of a mixture with at least a portion of (a).

**Revendications**

1. Procédé de préparation de modèles de plâtre pour la technique dentaire par traitement d'un modèle de plâtre, obtenu de manière connue, avec des esters polymérisables d'acide acrylique et/ou méthacrylique et des initiateurs de polymérisation, ainsi que par polymérisation des monomères, caractérisé en ce que l'on imprègne le modèle de plâtre avec

(a) au moins un ester photo-polymérisable de l'acide acrylique et/ou méthacrylique,

(b) au moins un initiateur de photo-polymérisation, et

(c) éventuellement au moins un activeur de photo-polymérisation,

(a) (b) et (c) étant présents à l'état liquide ou dissous, et l'on irradie le modèle de plâtre imprégné avec une lumière appropriée pour le démarrage de la photo-polymérisation par le photo-initiateur.

2. Procédé selon la revendication 1, caractérisé en ce que (b) et/ou (c) sont mis en réaction sous forme de mélange avec au moins une partie de (a).